# DEMANDE DE BREVET EUROPEEN

(11) **EP 1 647 261 A1**
(43) Date de publication de la demande: **19.04.2006**
(21) Numéro de dépôt: 05292140.0
(22) Date de dépôt: 13.10.2005
(51) Int. Cl.: A61K 8/40, A61Q 5/06, A61K 8/20, A61K 8/24, A61K 8/55, A61K 8/58, A61K 8/892

(54) **Composition pour le traitement des matières kératiniques à base de monomères électrophiles et de sels**

(30) Priorité: 13.10.2004 FR 0410811
(71) Demandeur: L'OREAL, 75008 Paris (FR)
(72) Inventeur: Vic, Gabin, 60280 Venette (FR)
(74) Mandataire: Casalonga, Axel

(57) **Abrégé**

La présente demande a pour objet des compositions cosmétiques, notamment pour le traitement des matières kératiniques, et notamment des fibres kératiniques telles que les cheveux, comprenant, dans un milieu cosmétiquement acceptable, au moins un monomère électrophile, et au moins un sel organique ou minéral particulier.

## Description

La présente invention concerne des compositions pour le traitement des matières kératiniques, et notamment des fibres kératiniques telles que les cheveux, à base de monomères polymérisables in situ, d'un milieu cosmétiquement acceptable, et de sels, ainsi que le procédé de traitement cosmétique correspondant.

Par "matières kératiniques", on entend de préférence les fibres kératiniques, et encore de préférence les cheveux.

On utilise généralement des compositions cosmétiques à base de silicones ou de polymères ayant une forte affinité pour les matières kératiniques, et notamment les cheveux, en vue de modifier leurs propriétés superficielles, notamment pour les conditionner et leur apporter de la brillance.

Il est généralement nécessaire de renouveler ces traitements dans la mesure où les agents de conditionnement ont tendance à s'éliminer, notamment aux shampoings.

Il est théoriquement possible d'accroître la rémanence du dépôt de polymères en effectuant directement une polymérisation radicalaire de certains monomères au niveau des cheveux.

Toutefois, les traitements ainsi obtenus sont inacceptables au point de vue cosmétique. On constate généralement une forte dégradation de la fibre liée probablement aux amorceurs de polymérisation et les cheveux traités sont difficilement démêlables, si bien qu'aujourd'hui, on cherche à obtenir des compositions cosmétiques permettant de conférer de la douceur et une brillance durable à la chevelure.

La demanderesse vient de découvrir de manière surprenante qu'il était possible d'obtenir un revêtement plus ou moins dur conférant aux matières kératiniques une brillance améliorée et durable, en mettant en oeuvre un mélange de monomères électrophiles, tels qu'ils sont décrits dans la demande FR 2840208, d'un milieu cosmétiquement acceptable et d'un sel organique ou minéral.

La demanderesse a plus particulièrement constaté qu'en appliquant une composition à base de tels monomères, d'un milieu cosmétiquement acceptable et d'un sel organique ou minéral sur les fibres kératiniques, il se forme in situ un revêtement lubrifiant et brillant rémanent.

Selon leur nature et leur concentration, les sels ajoutés renforcent la rigidité du gainage ou au contraire le rendent plus adhérent. Un tel gainage peut apporter, en plus des propriétés de brillance et de douceur, des propriétés coiffantes rémanentes marquées.

Par ailleurs, la demanderesse a constaté de façon surprenante que les cheveux restaient parfaitement individualisés et pouvaient être coiffés sans problème, et que le conditionnement et la brillance de la fibre étaient rémanents aux shampooings.

L'invention a donc pour premier objet une composition cosmétique, pour le traitement des matières kératiniques, et notamment des fibres kératiniques telles que les cheveux, comprenant, dans un milieu cosmétiquement acceptable, au moins un monomère électrophile, et au moins un sel, organique ou minéral, non réducteur, et de point de fusion supérieur à 60°C, sous réserve que si le sel est un sel organique ou minéral dont l'anion contient au moins un atome de soufre, alors la tension de surface de ce sel à la concentration de 1% en poids dans l'eau est supérieure ou égale à 60 mN/m.

L'invention a également pour objet un procédé mettant en oeuvre une composition utilisée dans le cadre de ce traitement.

Elle a également pour objet l'utilisation des compositions pour le traitement des matières kératiniques, et notamment des fibres kératiniques telles que les cheveux.

Elle a enfin pour objet un kit comprenant une première composition contenant au moins un monomère électrophile (présent à des teneurs pouvant être comprise entre 0,5 et 50% du poids de la première composition) et éventuellement au moins un inhibiteur de polymérisation anionique et/ou radicalaire (présent à des teneurs pouvant être comprise entre 10 ppm et 5% du poids de la première composition), ainsi qu'une deuxième composition comprenant dans un milieu cosmétiquement acceptable au moins un sel organique ou minéral tel que défini ci-dessus (présent à des teneurs pouvant être comprise entre 0,001 et 5% du poids de la deuxième composition).

D'autres objets de l'invention apparaîtront à la lecture de la description et des exemples qui suivent.

Par monomère électrophile, on entend un monomère capable de polymériser par polymérisation anionique en présence d'un agent nucléophile tel que par exemple, les ions hydroxyles (OH-) contenus dans l'eau.

Par polymérisation anionique, on entend le mécanisme défini dans l'ouvrage "Advanced Organic Chemistry", Third Edition de Jerry March, pages 151 à 161.

Le ou les monomères électrophiles présents dans la composition de l'invention peuvent être choisis parmi :
- les dérivés benzylidene malononitrile (A), le 2-(4-chloro-benzylidene)-malononitrile (A1) le 2-cyano-3-phényl acrylate d'éthyle (B), le 2-cyano-3-(4-chloro-phényl) acrylate d'éthyle (B1) décrits dans Sayyah, *J. Polymer Research,* 2000, p97 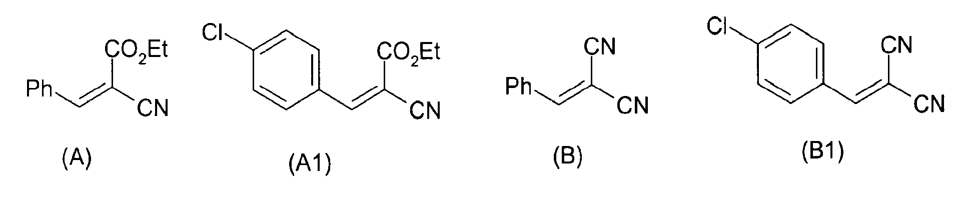
- les dérivés de méthylidenemalonates comme :
   - le 2-méthylene-malonate de diéthyle (C) par Hopff, *Makromoleculare Chemie,* 1961, p95, De Keyser, *J. Pharm. Sci,* 1991, p67 et Klemarczyk, *Polymer,* 1998, p 173 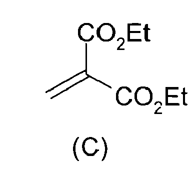
   - le 2-éthoxycarbonylméthyleneoxycarbonyl acrylate d'éthyle (D) par Breton, *Biomaterials,* 1998, p271 et Couvreur, *Pharmaceutical Research,* 1994, p 1270. 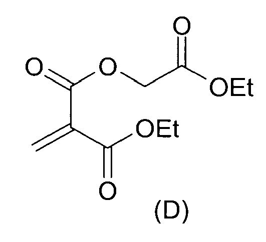
- les dérivés itaconate et itaconimide comme :
   - l'itaconate de diméthyle (E) par Bachrach, *European Polymer Journal,* 1976, p563 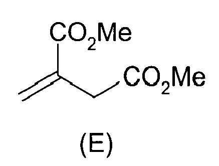
   - N-butyl itaconimide (F), N-(4-tolyl) itaconimide (G), N-(2-ethylphenyl) itaconimide (H), N-(2,6-diethylphenyl) itaconimide (I) par Wanatabe, *J.Polymer Science : Part A : Polymer chemistry,* 1994, p2073 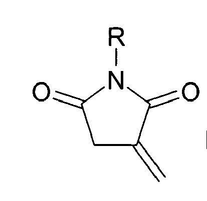 R= Bu (F), 4-tolyl (G), 2-ehylphenyl (H), 2,6-diethyphenyl (I)
- les dérivés α-(methylsulfonyl)acrylates de méthyle (K), α-(methylsulfonyl)acrylates d'éthyle (L), α-(tert-butylsulfonyl)acrylates de méthyle (M), α-(methylsulfonyl)acrylates de tert-butyle (N), α-( tert-butylsulfonyl)acrylates de tert-butyle (O), par Gipstein, *J. Org. Chem,* 1980, p1486 et
- les dérivés 1,1-bis-(methylsulfonyl)ethylene (P), 1-acetyl-1-methyl sulfonyl ethylene (Q), α-(methylsulfonyl) vinyl sulfonate de methyle (R), α-methylsulfonylacrylonitrile (S) par Shearer, US patent US2748050. 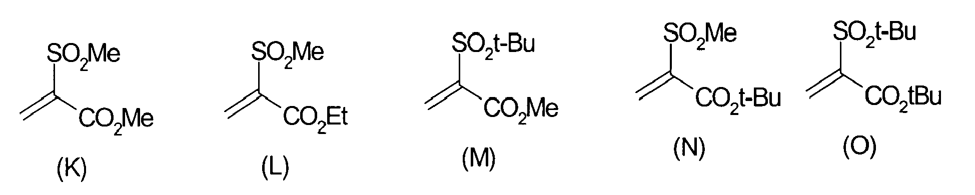 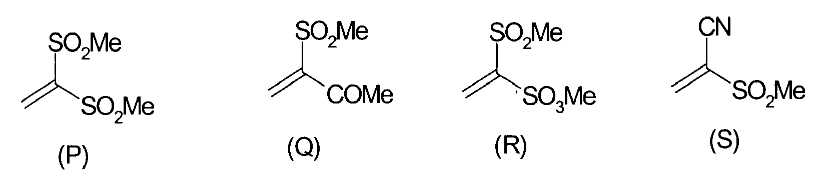
- les dérivés méthyl vinyl sulfone (T) et phényl vinyl sulfone (U) par Boor, *J.Polymer Science,* 1971, p249 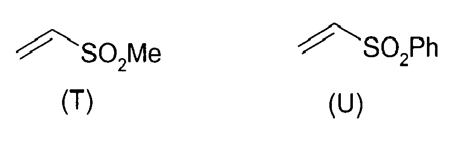
- le dérivé phényl vinyl sulfoxide (V) par Kanga, *Polymer preprints (ACS, Divison of Polymer Chemistry),* 1987, p322 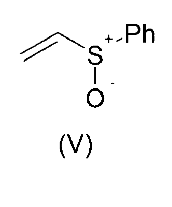
- le dérivé 3-methyl-N-(phenylsulfonyl)-1-aza-1,3-butadiene (W) par Bonner, *Polymer Bulletin,* 1992, p517 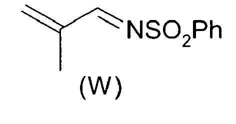
- les dérivés acrylates et acrylamides comme :
   - N-propyl-N-(3-triisopropoxysilylpropyl)acrylamide (X) et N-propyl-N-(3-triethoxysilylpropyl)acrylamide (Y) par Kobayashi, *Journal of Polymer Science, Part A: Polymer Chemistry,* 2005, p2754. 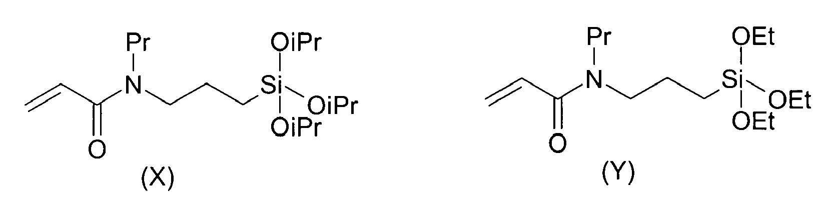
   - 2-hydroxyethyl acrylate (Z) et 2-hydroxyethyl méthacrylate (AA) par Rozenberg, *International Journal of Plastics Technology,* 2003, p17 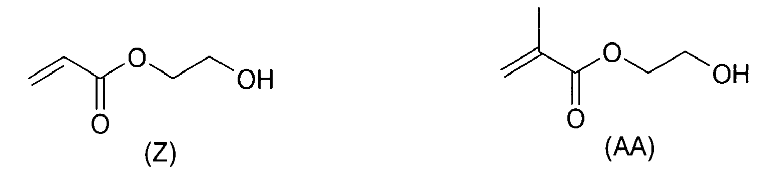
   - N-butyl acrylate (AB) par Schmitt, *Macromolecules,* 2001, p2115
   - Tert-butyl acrylate (AC) par Ishizone, *Macromolecules,* 1999, p955.

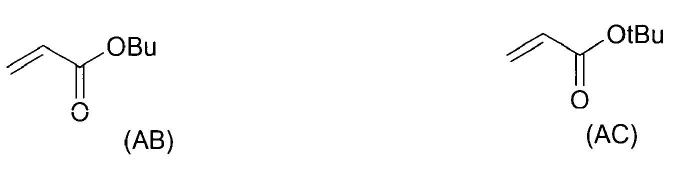

Le monomère électro-attracteur utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères présentant au moins deux groupes électro-attracteurs, on peut citer les monomères de formule (I) : 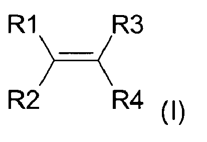 dans laquelle :
R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR,
   - OH, et les atomes d'halogène,
   - un résidu polyorganosiloxane modifié ou non,
   - un groupement polyoxyalkylène,
R₃ et R₄ désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N, -COOH, -COOR, -COSR, -CONH₂, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C1-C4, les groupements aryle tels que phényle, ou les groupements aryloxy tels que phénoxyloxy,
R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi ―OR', - COOR', -COR', -SH, -SR', -OH, les atomes d'halogène, ou un résidu de polymère pouvant être obtenu par polymérisation radicalaire ou par polycondensation ou par ouverture de cycle, R' désignant un groupe alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)n-(CF₂)m-CF₃ ou -(CH₂)n-(CF₂)m-CHF₂ avec n=1 à 20 et m= 1 à 20.

Les substituants R1 à R4 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoïques, quinoniques, méthiniques, cyanométhiniques et triarylméthane.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et de type esters gras.

Parmi les monomères précédemment cités, sont préférés les monomères de la famille des cyanoacrylates et leurs dérivés de formule (II) : 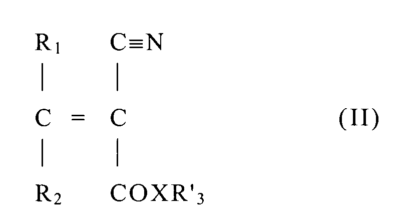 X désignant NH,S,O,
R₁ et R₂ ayant les mêmes significations que précédemment,
R'₃ pouvant désigner un atome d'hydrogène ou un radical R tel que défini pour la formule (I).

De préférence, X désigne O.

A titre de composés de formule (II), on peut citer les monomères :
a) appartenant à la famille des 2-cyanoacrylates de polyfluoroalkyle en C₁-C₂₀ tels que :
   l'ester 2,2,3,3-tétrafluoropropylique de l'acide 2-cyano-2-propénoïque de formule : 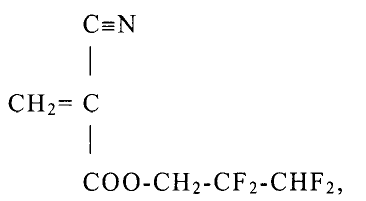
   ou encore l'ester 2,2,2-trifluoroéthylique de l'acide 2-cyano-2-propénoïque de formule : 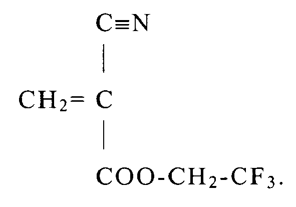
b) les cyanoacrylates d'alkyle en C₁-C₁₀ ou d'(alcoxy en C₁₋C₄)(alkyle en C₁-C₁₀).
   On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b).

Les monomères le plus particulièrement préférés sont ceux de formule III et leurs mélanges : 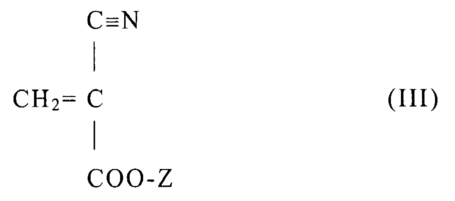 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Par milieu cosmétiquement acceptable, on entend un milieu compatible avec les matières kératiniques telles que les cheveux.

Le milieu cosmétiquement acceptable est de préférence anhydre. On entend par « milieu anhydre », un milieu contenant moins de 1 % en poids d'eau par rapport au poids total de la composition.

Le milieu cosmétiquement acceptable est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ; les cires ; ou encore des composés organiques tels que des alcanes en C₅-C₁₀, l'acétone, la méthyléthylcétone, les esters d'acides en C₁-C₂₀ et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras en C₁₀-C₃₀ tels que l'alcool laurique, l'alcool cétylique, l'alcool stéarylique, l'alcool béhénylique ; les acides gras en C₁₀-C₃₀ tels que l'acide laurique, l'acide stéarique ; les amides gras en C₁₀-C₃₀ tels que la diéthanolamide laurique, les esters d'alcools gras en C₁₀-C₃₀ tels que les benzoates d'alcool gras en C₁₀-C₃₀ et leurs mélanges.

De préférence, les composés organiques sont choisis parmi les composés liquides à la température de 25°C et sous 105 Pa (760mm de Hg).

Les compositions mises en oeuvre conformément à l'invention ont généralement une concentration en monomère électrophile selon l'invention comprise entre 0,001 et 80 % en poids, et plus particulièrement entre 0,1 et 40 % et encore plus préférentiellement entre 1 et 20 % en poids par rapport au poids total de la composition.

On peut également introduire dans les compositions, des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges. Les groupements alkyle désignent de préférence des groupements ayant 1 à 6 atomes de carbone.

On peut aussi utiliser des acides organiques ou minéraux, ces derniers ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique.

La quantité d'inhibiteur peut aller de 10 ppm à 20%, et plus préférentiellement de 10 ppm à 5% et encore plus préférentiellement de 10 ppm à 1 % en poids par rapport au poids total de la composition.

Les compositions conformes à l'invention peuvent également contenir au moins un agent utilisé habituellement en cosmétique, tel que, par exemple, des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, et des agents nacrants, des gaz propulseurs, des épaississants minéraux ou organiques tels que le benzylidène sorbitol, les N acylaminoacides. Ces agents peuvent être éventuellement encapsulés. La capsule peut être de type polycyanoacrylate.

On entend par sel organique ou minéral tout composé non-polymérique formé par réaction d'un acide organique ou minéral sur une base organique ou minérale.

De préférence, les sels de l'invention sont solubles dans le milieu contenant le ou les monomères électrophiles. Par soluble, on entend au sens de la présente invention la formation d'une solution macroscopiquement homogène.

De préférence, si le sel est issu d'une base organique, sa tension de surface à 1% dans l'eau est supérieure à 60 mN/m.

De façon non exhaustive, on peut citer les sels minéraux tels que le chlorure de sodium, le chlorure de magnésium, le sulfate de cuivre.

De façon non exhaustive, on peut aussi citer les sels organiques formés par réaction d'un anion choisi parmi les anions suivants : phosphates, borates, silicates, bicarbonates, carbonates, chlorates, nitrates, silicates, sulfonate, contenant une ou plusieurs chaînes hydrocarbonées en C₁-C₃₀, saturées ou insaturées, cycliques ou non cycliques, éventuellement substituées par un ou plusieurs groupements choisis parmi les groupements hydroxy, alcoxy en C₁-C₄, halogène,
avec un cation choisi parmi les cations suivants :
potassium, sodium, strontium, cadmium, calcium, ammonium (tel que tétra-alkyle ammonium ou aryle ammonium), phosphonium, barium, lithium, magnésium.

On peut citer à titre de sels organiques les mono et dibutyl phosphates de sodium, les mono et diéthyl phosphate de sodium.

Le sel organique ou minéral peut être présent dans la composition à des teneurs comprises entre 0,001 et 70% en poids, de préférence entre 0,01 et 20%, et encore de préférence entre 0,1 et 10% en poids du poids total de la composition.

Le procédé de traitement des cheveux conforme à l'invention consiste à appliquer la composition décrite ci-dessus sur les matières kératiniques, et en particulier en présence d'un agent nucléophile avec ou sans chauffage.

De préférence, cet agent nucléophile est l'eau. Cette eau peut être apportée par une humidification préalable.

Il est également possible, afin de moduler la cinétique de réaction, de préalablement humidifier les matières kératiniques à l'aide d'une solution aqueuse dont le pH a été ajusté à l'aide d'une base, d'un acide ou d'un mélange acide/base. L'acide et/ou la base peuvent être inorganique ou organique.

Ces deux opérations peuvent aussi être effectuées après application de la composition.

Il est également possible de moduler la cinétique de polymérisation par voie anionique en pré-imprégnant les matières kératiniques à l'aide d'un agent nucléophile. L'agent nucléophile peut être utilisé pur, en solution, sous forme d'une émulsion, ou être encapsulé.

Les agents nucléophiles susceptibles d'initier la polymérisation anionique sont des systèmes connus en eux-mêmes, capables de générer un carbanion au contact d'un agent nucléophile, tels que les ions hydroxyles contenus dans l'eau. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C≡C⁻, RS⁻, SH, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O Ph représentant le groupe phényle ; Ar représentant un groupe aryle et R représentant un groupe alkyle en C₁-C₁₀.

De préférence, l'agent nucléophile est l'eau. Cette eau peut être apportée par une humidification préalable.

Il est également possible, afin de moduler la cinétique de réaction, de préalablement humidifier la fibre à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant la fibre à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie de la fibre par transformation chimique de la matière kératinique.

A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter la fibre avec tous types de polymères, ou réaliser un traitement capillaire avant application de la composition de l'invention, comme une coloration directe ou d'oxydation, une permanente ou encore un défrisage.

L'application des compositions peut être suivie ou non d'un rinçage. Ces compositions peuvent se présenter sous des formes diverses, telles que de lotion, de spray, de mousse et être appliquées sous forme de shampooing ou d'après-shampooing.

Le procédé peut comprendre une étape d'application sur les matières kératiniques d'au moins un sel tel que défini ci-dessus et une étape d'application sur les matières kératiniques d'au moins un monomère électrophile, l'ordre des étapes étant indifférent.

Dans un mode de réalisation particulier, l'application du ou des sels est réalisée avant l'application du ou des monomères électrophiles.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence de 20 à 80°C, ce qui n'empêche pas de terminer l'application par un séchage.

L'invention concerne également l'utilisation des compositions décrites ci-dessus pour le traitement des cheveux, et en particulier pour obtenir une brillance améliorée et durable.

Les exemples qui suivent sont destinés à illustrer l'invention, sans toutefois présenter un caractère limitatif.

### Exemple 1

### crème 1 de brillance (sans sel)

| 2-cyanoacrylate de n-octyle (1) | 5% m.a. |
|---|---|
| cyclopentasiloxane | 47.4% |
| cyclopentasiloxane dimethicone copolyol | 47.4% |
| parfum | 0.2% |

| | |
|---|---|
| Les pourcentages sont exprimés en poids | |
| m. a.: matière active. | |
| (1) RITE LOK CON895, commercialisé par la société CHEMENCE | |

### crème 2 de brillance selon l'invention (avec sel)

| 2-cyanoacrylate de n-octyle (1) | 5% m.a. |
|---|---|
| cyclopentasiloxane | 46.65% |
| cyclopentasiloxane dimethicone copolyol | 46.65% |
| Diéthyl phosphate de sodium | 1.5% |
| parfum | 0.2% |

| | |
|---|---|
| Les pourcentages sont exprimés en poids | |
| m. a.: matière active. | |
| (1) RITE LOK CON895, commercialisé par la société CHEMENCE | |

### Mode d'application

Des mèches constituées de 2,7 g de cheveux sensibilisés sont humidifiées à l'aide d'1 ml d'eau par mèche. 2 g des compositions décrites ci-dessus sont appliqués sur ces mèches humidifiées. Après application, les mèches de cheveux sont séchées au casque pendant 30 minutes à 40°C.

Pour chaque mèche, le toucher et la brillance des cheveux sont évalués par un panel de 10 personnes. Une mèche vierge de même nature est utilisée comme référence. L'évaluation tactile et visuelle des diverses mèches de cheveux est répétée avec la même procédure après avoir réalisé successivement 5 shampooings commercialisés sous la dénomination DOP camomille.

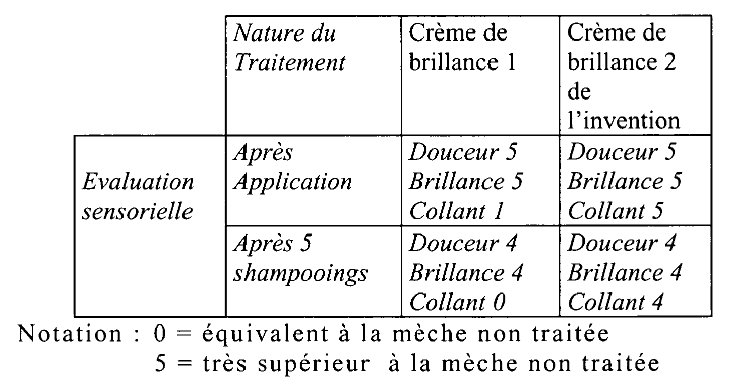

L'expérience montre que les propriétés cosmétiques (douceur, brillance et collant apportés par la composition de l'invention sont supérieurs aux propriétés cosmétiques apportées par la crème 1, juste après application des compositions. De plus, après réalisation de 5 shampooings, la douceur, la brillance et le collant apportés par la composition de l'invention sont conservés.

La composition de l'invention permet, après plusieurs shampooings, de maintenir la douceur, la brillance et le collant apportés à la chevelure et ceci sans qu'il soit nécessaire d'appliquer à nouveau la composition.

Il est à noter que les niveaux de brillance et de douceur sont plus élevés avec la composition de l'invention si celle-ci est appliquée sur des cheveux mouillés (mode d'application n°2).

### Exemple 2

Les pourcentages sont exprimés en poids

m. a . : matière active.

### crème de brillance

| methylheptylcyanoacrylate (1) | 5% m.a. |
|---|---|
| cyclopentasiloxane | 46.65% |
| cyclopentasiloxane dimethicone copolyol | 46.65% |
| Di-cétyl phosphate de sodium | 1.5% |
| parfum | 0.2% |

| | |
|---|---|
| (1)commercialisé par la société CHEMENCE | |

### Exemple 3

### crème de brillance

| ethoxyethylcyanoacrylate (1) | 5% m.a. |
|---|---|
| cyclopentasiloxane | 45.65% |
| cyclopentasiloxane dimethicone copolyol | 46.65% |
| Di-cétyl phosphate de sodium | 1.5% |
| Acide acétique | 1 % |
| parfum | 0.2% |

| | |
|---|---|
| (1) EO 460 commercialisé par la société Tong Shen | |

### Exemple 4

### crème de brillance

| butylcyanoacrylate (1) | 5% m.a. |
|---|---|
| cyclopentasiloxane | 45.65% |
| cyclopentasiloxane dimethicone copolyol | 46.65% |
| Acide acétique | 1 % |
| Di-cétyl phosphate de sodium | 1.5% |
| parfum | 0.2% |

| | |
|---|---|
| (1) B 60 commercialisé par la société Tong Shen | |

### Exemple 5

### crème de brillance

| ethylhexylcyanoacrylate (1) | 5% m.a. |
|---|---|
| cyclopentasiloxane | 46.65% |
| cyclopentasiloxane dimethicone copolyol | 46.65% |
| Di-cétyl phosphate de sodium | 1.5% |
| parfum | 0.2% |

| | |
|---|---|
| (1) O-60 commercialisé par la société Tong Shen | |

### Exemple 6

### crème de brillance

| methylheptylcyanoacrylate (1) | 4.5% m.a. |
|---|---|
| Ethylhexylcyanoacrylate (2) | 0.5% |
| cyclopentasiloxane | 46.65% |
| cyclopentasiloxane dimethicone copolyol | 46.65% |
| Di-cétyl phosphate de sodium | 1.5% |
| parfum | 0.2% |

| | |
|---|---|
| (1) commercialisé par la société Chemence | |
| (2) O-60 commercialisé par Tong Shen | |

### Exemple 7

### crème de brillance

| methylheptylcyanoacrylate (1) | 3.5% m.a. |
|---|---|
| butylcyanoacrylate (2) | 1.5% |
| cyclopentasiloxane | 46.65% |
| cyclopentasiloxane dimethicone copolyol | 46.65% |
| Di-cétyl phosphate de sodium | 1.5% |
| parfum | 0.2% |

| | |
|---|---|
| (1) commercialisé par la société Chemence | |
| (2) B-60 commercialisé par Tong Shen | |

## Revendications

1. Composition cosmétique, **caractérisée en ce qu'**elle comprend, dans un milieu cosmétiquement acceptable, au moins un monomère électrophile, et au moins un sel organique ou minéral non réducteur, de point de fusion supérieure à 60°C, sous réserve que si le sel est un sel organique dont l'anion contient au moins un atome de soufre, alors la tension de surface de ce sel à la concentration de 1% en poids dans l'eau est supérieure ou égale à 60 mN/m.

2. Composition selon la revendication 1, **caractérisée en ce que** le ou les monomères électrophiles répondent à la formule : 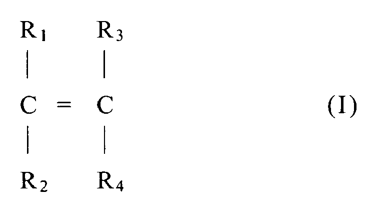 dans laquelle :
R₁ et R₂ désignent chacun, indépendamment l'un de l'autre, un groupe peu ou non électro-attracteur choisi parmi :
- un atome d'hydrogène,
- un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
- un résidu polyorganosiloxane modifié ou non,
- un groupement polyoxyalkylène,
R₃ et R₄ désignent chacun, indépendamment l'un de l'autre, un groupe électro-attracteur choisi parmi les groupements -N(R)₃⁺, - S(R)₂⁺, -SH₂⁺, -NH₃⁺, -NO₂, -SO₂R, -C≡N, -COOH, -COOR, -COSR, - CONH₂, -F, -Cl, -Br, -I, ―OR, -COR, -SH, -SR, -OH, les groupes alcényle linéaires ou ramifiés, les groupes alcynyle linéaires ou ramifiés, les groupements mono- ou polyfluoroalkyle en C1-C4, les groupements aryle et aryloxy,
R désigne un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire ou par polycondensation ou par ouverture de cycle, R' désignant un radical alkyle en C₁-C₁₀.

3. Composition selon la revendication 2, **caractérisée en ce que** le ou les monomères électrophiles sont choisis parmi les composés de formule : 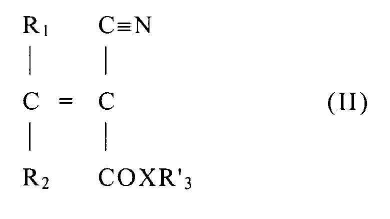 X désigne NH, S, O,
R₁ et R₂ sont tels que définis dans la revendication 2,
R'₃ désigne un atome d'hydrogène ou un radical R tel que défini dans la revendication 2.

4. Composition selon la revendication 3, **caractérisée en ce que** le ou les monomères sont choisis parmi les 2-cyanoacrylates de polyfluoroalkyle en C₁-C₂₀, les cyanoacrylates d'alkyle en (C₁-C₁₀) ou d'(alcoxy en C₁-C₄)(alkyle en C₁-C₁₀).

5. Composition selon la revendication 4, **caractérisée en ce que** le ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle le 2-cyanoacrylate de n-octyle et le cyanoacrylate d'iso-amyle.

6. Composition selon la revendication 3, **caractérisée en ce que** le ou les monomères électrophiles répondent à la formule (III) : 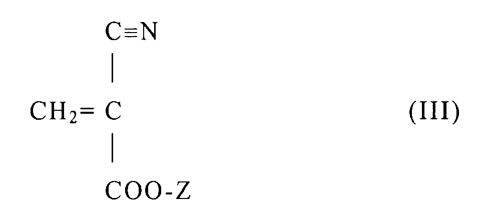 dans laquelle Z-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

7. Composition selon l'une quelconque des revendications précédentes, **caractérisée en ce que** le monomère est présent dans la composition à des teneurs comprises entre 0,001 et 80% en poids, de préférence entre 0,1 et 40%, et encore de préférence entre 1 et 20% en poids du poids total de la composition.

8. Composition selon l'une quelconque des revendications précédentes, **caractérisée en ce que** les monomères sont fixés de façon covalente sur des supports tels que des polymères, des oligomères ou des dendrimères.

9. Composition selon l'une quelconque des revendications précédentes, **caractérisée en ce que** le milieu cosmétiquement acceptable est anhydre.

10. Composition selon la revendication 9, **caractérisé en ce que** le milieu cosmétiquement acceptable est choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges.

11. Composition d'une composition selon l'une quelconque des revendications précédentes, **caractérisée en ce que** la composition comprend des inhibiteurs de polymérisation.

12. Composition selon la revendication 10, **caractérisée en ce que** les inhibiteurs sont des inhibiteurs de polymérisation anioniques et/ou radicalaires.

13. Composition selon la revendication 10, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges.

14. Composition selon l'une quelconque des revendications 11 à 13, **caractérisée en ce que** les inhibiteurs de polymérisation sont présents en des quantités allant de 10 ppm à 20%, de préférence allant de 10 ppm à 5%, et plus préférentiellement entre 10 ppm et 1% en poids du poids total de la composition.

15. Composition selon l'une quelconque des revendications précédentes, **caractérisée en ce que** le sel est un sel minéral choisi parmi le chlorure de sodium, le chlorure de magnésium, le sulfate de cuivre.

16. Composition selon l'une quelconque des revendications 1 à 14, **caractérisée en ce que** le sel est un sel organique formés par réaction d'un anion choisi parmi les anions suivants : phosphates, borates, silicates, bicarbonates, carbonates, chlorates, nitrates, silicates, sulfonate, contenant une ou plusieurs chaînes hydrocarbonées en C1-C30, saturées ou insaturées, cycliques ou non cycliques, éventuellement substituées par un ou plusieurs groupements choisis parmi les groupements hydroxy, alcoxy en C1-C4, halogène, avec un cation choisi parmi les cations suivants : potassium, sodium, strontium, cadmium, calcium, ammonium (tel que tétra-alkyle ammonium ou aryle ammonium), phosphonium, barium, lithium, magnésium.

17. Composition selon la revendication 16, **caractérisée en ce que** le sel organique est choisi parmi les mono et dibutyl phosphate de sodium, les mono et diéthyl phosphate de sodium.

18. Composition selon l'une quelconque des revendications précédentes, **caractérisée en ce que** le sel organique ou minéral est présent dans la composition à des teneurs comprises entre 0,001 et 70% en poids, de préférence entre 0,01 et 20%, et encore de préférence entre 0,1 et 10% en poids du poids total de la composition.

19. Composition selon l'une quelconque des revendications précédentes, **caractérisée en ce que** la composition comprend en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensioactifs anioniques, cationiques, non ioniques ou amphotères, les polymères fixants ou non, les polyols, les protéines, les vitamines, les colorants directs ou d'oxydation, et les agents nacrants.

20. Composition selon la revendication 19, **caractérisée en ce que** l'agent est encapsulé.

21. Composition selon l'une quelconque des revendications précédentes, **caractérisée en ce que** la composition est sous forme de lotion, de spray ou de mousse.

22. Procédé de traitement des matières kératiniques, **caractérisé en ce qu'**il comprend une étape d'application sur les matières kératiniques d'au moins un sel tel que défini dans la revendication 1 et une étape d'application sur les matières kératiniques d'au moins un monomère électrophile.

23. Procédé selon la revendication 22, **caractérisée en ce que** l'application du ou des sels est réalisée avant l'application du ou des monomères électrophiles.

24. Procédé de traitement des matières kératiniques, **caractérisé en ce qu'**on applique sur les matières kératiniques une composition utilisée dans l'une quelconque des revendications 1 à 21, en présence d'un agent nucléophile.

25. Procédé selon la revendication 24, **caractérisé en ce que** l'agent nucléophile est choisi parmi les composés moléculaires, les oligomères, les dendrimères ou polymères possédant des fonctions nucléophiles choisies parmi: R₂N⁻, NH₂⁻, Ph₃C⁻, R₃C⁻, PhNH⁻, pyridine, ArS⁻, R-C^{≡}C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle, Ar représentant un groupe aryle et R représentant un groupe aryle en C₁-C₁₀.

26. Procédé selon la revendication 24, **caractérisé en ce que** l'agent nucléophile est l'eau.

27. Procédé selon l'une quelconque des revendications 24 à 26, **caractérisé en ce que** l'on applique la composition sur les matières kératiniques préalablement humidifiées à l'aide d'une solution aqueuse dont le pH a été ajusté à l'aide d'une base, d'un acide ou d'un mélange acide/base.

28. Procédé selon l'une quelconque des revendications 24 à 26, **caractérisé en ce que** les matières kératiniques sont pré-imprégnées à l'aide d'un agent nucléophile autre que l'eau.

29. Procédé selon l'une quelconque des revendications 24 à 26, **caractérisé en ce que** les matières kératiniques sont préalablement réduites avant application de la composition.

30. Procédé selon la revendication 29, **caractérisé en ce que** l'agent réducteur est choisi parmi le thiosulfate de sodium anhydre, le métabisulfite de sodium en poudre, la thiourée, le sulfite d'ammonium, l'acide thioglycolique, l'acide tiolactique, le thiolactate d'ammonium, le mono-thioglycolate de glycérol, le thioglycolate d'ammonium, le thioglycérol, l'acide 2,5-dihydroxybenzoique, le di-thioglycolate de diammonium, le thioglycolate de strontium, le thioglycolate de calcium, le formo-sulfoxylate de zinc, le thioglycolate d'isooctyle, la di-cystéine, le thioglycolate de monoéthanolamine.

31. Procédé selon l'une des revendications 24 à 30, **caractérisé en ce que** la composition comprend en outre un polymère choisi parmi le poly(méthacrylate de méthyle) et les copolymères à base de cyanoacrylates.

32. Procédé selon l'une des revendications 24 à 31, **caractérisé en ce que** l'application de la composition est suivie d'un rinçage.

33. Utilisation d'une composition selon l'une quelconque des revendications 1 à 21 pour le traitement des matières kératiniques, et notamment des fibres kératiniques telles que les cheveux.

34. Utilisation selon la revendication 33 pour obtenir une brillance des cheveux améliorée et durable.

35. Kit comprenant une première composition contenant au moins un monomère électrophile et éventuellement au moins un inhibiteur de polymérisation anionique et/ou radicalaire, ainsi qu'une deuxième composition comprenant dans un milieu cosmétiquement acceptable au moins un sel organique ou minéral non réducteur, de point de fusion supérieure à 60°C, sous réserve que si le sel est un sel organique dont l'anion contient au moins un atome de soufre, alors la tension de surface de ce sel à la concentration de 1 % en poids dans l'eau est supérieure ou égale à 60 mN/m.
